# EUROPEAN PATENT SPECIFICATION

(11) **EP 2 537 827 B1**
(45) Date of publication and mention of the grant of the patent: **11.06.2014**
(21) Application number: 11360025.8
(22) Date of filing: 24.06.2011
(51) Int. Cl.: C07C 227/22, C07C 229/30, C07D 207/267, C07D 207/273, A61K 31/197, A61P 25/08

(54) **Process for preparing 4-amino-5-hexenoic acid from succinimide**
Verfahren zur Herstellung von 4-Amino-5-Hexensäure aus Succinimid
Procédé pour la préparation d'acide 4-amino-5-hexénoïque du succinimide

(43) Date of publication of application: 26.12.2012
(73) Proprietor: Targeon SAS, 75004 Paris (FR)
(72) Inventor: Duclos, Marie-Christine, 69100 Villeurbanne (FR); Bienaymé, Hugues, 69360 Saint-Symphorien d'Ozon (FR); Popowycz, Florence, 69100 Villeurbanne (FR); Lemaire, Marc, 69100 Villeurbanne (FR)
(74) Representative: Barny, Luc

(56) References cited:
- EP-A1- 0 116 257
- EP-A1- 0 427 197
- US-B1- 6 713 497
- TOJA, E. ET AL.: "Amnesia-reversal activity of a series of 5-alkoxy-1-arylsulfonyl-2-pyrrolidinones", EUROPEAN JOURNAL OF MEDICINAL CHEMISTRY, vol. 26, no. 4, 1991, pages 403-413, XP023870913, ISSN: 0223-5234, DOI: 10.1016/0223-5234(91)90101-R [retrieved on 1991-06-01]
- COULTON, S. ET AL.: "Synthesis of gamma-lactam analogues of 1-acetoxy carbapenem derivatives", TETRAHEDRON LETTERS, vol. 31, no. 47, 1990, pages 6923-6926, XP55028874,
- WEI, Z.-Y. ET AL.: "Asymmetric synthesis of both enantiomers of Vigabatrin: An approach using methionine as the chiral pool", TETRAHEDRON, vol. 50, no. 19, 1994, pages 5569-5578, XP55028879,
- GHEORGHE, A. ET AL.: "Synthesis of Functionalized Pyrrolidin-2-ones and ( S )-Vigabatrin from Pyrrole", THE JOURNAL OF ORGANIC CHEMISTRY, vol. 71, no. 5, 2006, pages 2173-2176, XP55028878, ISSN: 0022-3263, DOI: 10.1021/jo0524472

## Description

The present invention relates to a new and competitive process for the preparation of 4-amino-5-hexenoic acid and intermediates thereof. This compound is a well-known anti-epileptic drug for which several syntheses have been developed, all of them presenting drawbacks. The new preparation process according to the present invention is very competitive from an economical point of view and is adapted to industrial scale preparation of 4-amino-5-hexenoic acid.

The compound 4-amino-5-hexenoic acid of general formula C₆H₁₁NO₂, also called 4-aminohex-5-enoic acid and which international common denomination is Vigabatrin for "gamma-vinyl-aminobutyric acid" or "vinyl-GABA" was described for the first time in the patent US 3,960,927. Its chemical structure (I) is as follows:

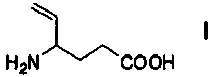

It is a well-known anti-epileptic agent and also the first intention treatment for West Syndrome mainly in Europe and Canada. It acts as an irreversible inhibitor of gamma-aminobutyric acid transaminase (GABA-T) the enzyme responsible for the catabolism of the inhibitory neurotransmitter gamma-aminobutyric acid (GABA) in the brain. Vigabatrin is commercially known as Sabri® in Belgium, Canada, Mexico, Switzerland, the United States of America and the United Kingdom, and Sabrilex® in Denmark. Vigabatrin is used as an adjunctive treatment in epilepsy and related syndromes, but as monotherapy for the treatment and/or prophylaxis of West syndrome. West syndrome (or Infantile spasms) is a rare, but devastating form of epileptic encephalopathy that occurs within the first year of life. West syndrome is characterized by peculiar seizures forms (repetitive flexor, extensor or flexor-extensor spasms), EEG hypsarrhythmia and poor seizure, and intellectual prognosis. The current drug administration in Europe is made once daily or twice daily, at a dose of 50 to 100 mg/kg. This dosage is high and involves a significant quantity of active substance. This high dosage generates a significant impact of the drug manufacturing cost on the daily treatment cost.

To date, several preparation processes for 4-amino-5-hexenoic acid are available among which the processes described in the patents US 4,178,463 US 4,621,145 US 4,235,778 and EP 0546906. As an example, 4-amino-5-hexenoic acid is currently produced at an industrial scale using the process described in the patents US 4,178,463, and US 4,235,778. In this process, 1,4-dichloro-2-butene is reacted with diethyl malonate under basic conditions to produce 2-vinyl cyclopropane-1,1-diethyldicarboxylate. 2-vinyl cyclopropane-1,1-diethyldicarboxylate is then reacted with ammonia under pressure to form 3-carboxamido-5-vinyl-2-pyrrolidone, which is further hydrolyzed under acidic conditions to form 4-amino-5-hexenoic acid (I). This process is hampered by several industrial drawbacks, including an expensive starting material (1,4-dichloro-2-butene) and a low overall yield that renders this process expensive from an economical point of view. Furthermore, 1,4-dichloro-2-butene is known to be a carcinogenic agent, which impose drastic safety measures when manipulated.

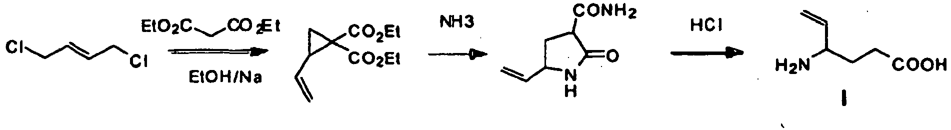

As another example, the patent EP 0546906 describes a process for preparing 4-amino-5-hexenoic acid that uses erythritol as starting material. During a six steps process involving thermal rearrangement and conversions, erythritol is transformed in 4-amino-5-hexenoic acid (Casara, P. Tetrahedron Lett. 1994, 35, 3049; patent EP 0546906) with an overall yield of 25%. This process is long and expensive due to reagent costs for an unsatisfying global yield of 4-amino-5-hexenoic acid. Other preparation processes with various starting material can also be mentioned:
- from homoserine lactone (Zhang, Z.; Ding, Y.-S.; Studenov, A. R.; Gerasimov, M. R.; Ferrieri, R. A. J. Label. Compd. Radiopharm. 2002, 45, 199) with a seven chemical steps synthesis and a very low global yield of 0,2%.
- from hydrocinnamaldehyde (Alcon, M.; Poch, M.; Moyano, A.; Pericàs, M. A.; Riera, A. Tetrahedron Asymm. 1997, 8, 2967) with an eleven steps synthesis and a poor global yield of 14%.
- from 3,4-epoxy-1-butene (Gnamm, C.; Franck, G.; Miller, N.; Stork, T.; Brödner, K.; Helmchen, G. Synthesis 2008, 3331) with a five steps synthesis and a global yield of 51%.
- from L-glutamic acid (Wei, Z.-Y.; Knaus, E. E. J. Org. Chem. 1993, 58, 1586) with an eight steps synthesis and a global yield of 38%; also for Frieben et al. (Frieben, W.; Gerhart, F.; patent US 4,621,145) with a nine steps synthesis and a global yield of 21%; and finally for Kwon et al. (Kwon, T. W.; Keusenkothen, P. F.; Smith, M. B. J. Org. Chem. 1992, 57, 6169) with a six steps synthesis and a global yield of 28%.
- from pyrrole (Gheorghe, A.; Schulte, M.; Reiser, O. J. Org. Chem. 2006, 71, 2173) with a ten steps synthesis and a poor global yield of 4%.
- from methionine (Wei, Z.-Y.; Knaus, E. E. Tetrahedron 1994, 50, 5569) with a six steps synthesis and a global yield of 34%.
- from pyrrolidi-2-one (Wambach, L.; Fischer, M.; patent US 4,874,865) with a three steps synthesis and a global yield of 42%.
- from 5-vinyl-2-pyrrolidinone for (Goralski et al., Tetrahedron Letters, 35, p. 847, 1994; patent EP 0427197) with a two steps synthesis and a global yield of 57 to 67%. This process only describes the last chemical steps of the whole synthesis.

All the above-mentioned prior art syntheses are either not amenable to industrial scale or impaired by low overall yields, making them economically ineffective. The only known process amenable to industrial scale seems to be the subject matter of the patents US 4,178,463, and US 4,235,778 as detailed above. There is a need for a new preparation process of 4-amino-5-hexenoic acid that is low expensive, with easily available starting material and reagents, and that is consequently amenable to an industrial scale in order to produce high quantity of drugs for the treatment of pathologies such as epilepsy.

The present invention is directed to a novel and economically competitive process for producing 4-amino-5-hexenoic acid of general formula (I), starting from readily available and inexpensive raw materials.

It is known from the work of Neipp et al. ( a) Neipp, C. E.; Martin, S. F. Tetrahedron Lett. 2002, 43, 1779; b) Neipp, C. E.; Martin, S. F. J. Org. Chem. 2003, 68, 8867) that glutarimide could be reduced by sodium borohydride in presence of hydrogen chloride in ethanol to form 6-ethyloxy-2-piperidone. Direct alkylation of 6-ethyloxy-2-piperidone with vinyl magnesium bromide was not observed. To obtain 6-vinyl-2-piperidone, the 6-ethyloxy-2-piperidone was first reacted with sodium phenyl sufinate under acidic condition to form 6-phenylsulfonyl-2-piperidone, and then reacted with vinyl magnesium bromide.

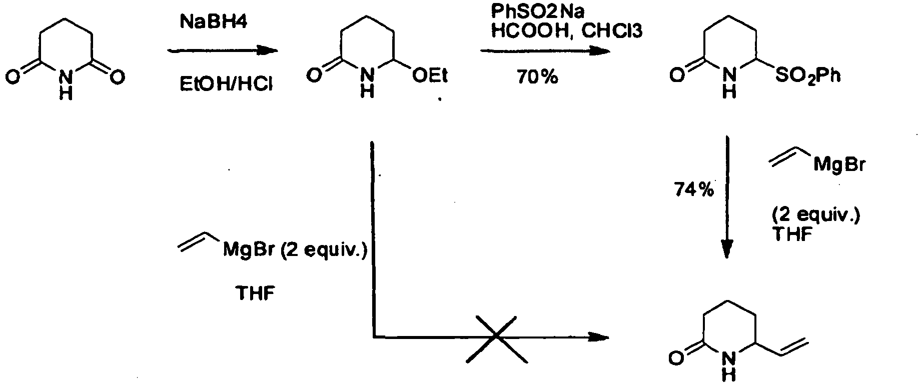

5-alkoxy-2-pyrrolidones (II) were reported to be synthesized by either irradiation of succinimide, although in low yield and selectivity (Ma, G.; Liu, H.; Zhang, W. J. Photochem. Photobiol. A : Chem. 2007, 187, 377), by electrolysis of pyroglutamic acid in methanol in high yield ( a)Iwasaki, T.; Horikawa, H.; Matsumoto, K.; Miyoshi, M. J. Org. Chem. 1979, 44, 1552; b)Tajima, T.; Kurihara, H.; Fuchigami, T. J. Am. Chem. Soc. 2007, 129, 6680; c)Mitzlaff, M.; Warning, K.; Rehling, H. Synthesis 1980, 315), although the later procedure is not easily transposable to industrial scale, or by sodium borohydride reduction of succinimide in variable yields (Toja et al. 1991 - Eur. J. Med. Chem., Vol 26, Issue 4, 1991, 415-422). 5-methoxy-pyrrolidone, equivalent to the compound of formula (II) wherein R=Me, was reported to be unstable and had to be manipulated below 20°C.

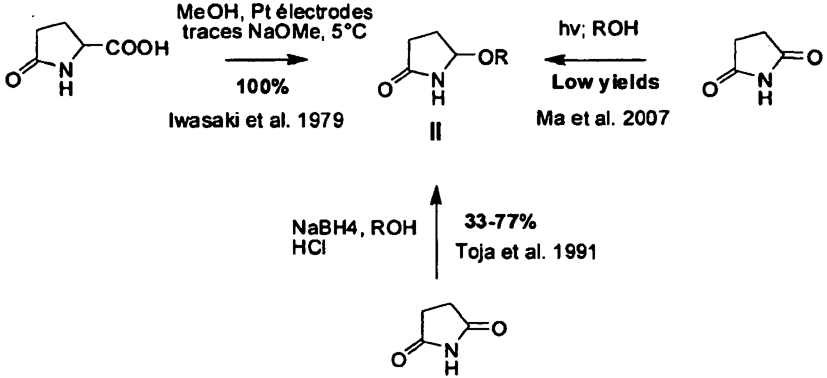

Furthermore, Coulton et al. (Coulton, S.; François, I.; Southgate, R. Tetrahedron Lett. 1990, 31, 6923) have shown that 5-methoxy-2-pyrrolidinone could be converted to 5-vinyl-2-pyrrolidinone by treatment with vinyl magnesium bromide.

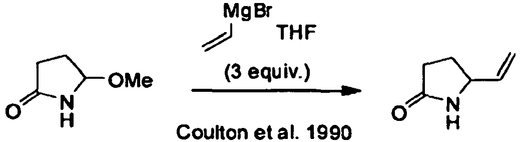

It is known from patents US 4,621,145 and US 6,090,979 that 5 -vinyl-2-pyrrolidinone can be hydrolyzed to 4-amino-5-hexenoic acid (I). The inventors have surprisingly discovered that the preparation process for 4-amino-5-hexenoic acid (I) consisting of the following steps: a) reduction of succinimide in an alcoholic solvent to form a 5-alkoxy-2-pyrrolidone intermediate (II), b) alkylation of said 5-alkoxy-2-pyrrolidone intermediate (II) with a vinyl magnesium halide reagent to form 5-vinyl-2-pyrrolidone (III) and c) hydrolysis of 5-vinyl-2-pyrrolidone (III) to 4-amino-5-hexenoic acid (I) is short in the way that it involves a low number of steps, and economically competitive in the way that the starting material and reagents are available and inexpensive.

The present invention is directed to a process for producing 4-amino-5-hexenoic acid using succinimide as raw material, characterized in that it comprises at least a first step a) consisting of reducing said succinimide with a hydride donor in an alcoholic solvent in presence of a protic acid to produce a 5-alkoxy-2-pyrrolidone intermediate. Subsequent steps for the preparation of 4-amino-5-hexenoic acid from 5-alkoxy-2-pyrrolidone can be realized by any method known by one skilled in the art. As an example, it can be mentioned: reacting the 5-alkoxy-2-pyrrolidone intermediate with a vinyl Grignard reagent in a suitable solvent to obtain 5-vinyl-2-pyrrolidone and hydrolyzing said 5-vinyl-2-pyrrolidone to 4-amino-5-hexenoic acid; or submitting the 5-alkoxy-2-pyrrolidone intermediate to a vinyl Grignard reagent in a suitable solvent and a basic reagent to form 5-vinyl-2-pyrrolidone and hydrolyzing said 5-vinyl-2-pyrrolidone to 4-amino-5-hexenoic acid.

Nevertheless, in a first embodiment, the present invention provides a process for preparing 4-amino-5-hexenoic acid (I), which comprises the following steps:
a) reducing succinimide with a hydride donor in an alcoholic solvent in presence of a protic acid to produce a 5-alkoxy-2-pyrrolidone intermediate (II);
b) reacting said 5-alkoxy-2-pyrrolidone intermediate (II) with at least 2 equivalents of a vinyl magnesium halide reagent (vinyl Grignard reagent) in a suitable solvent, to form 5-vinyl-2-pyrrolidone (III); and
c) hydrolyzing said 5-vinyl-2-pyrrolidone (III) to 4-amino-5-hexenoic acid (I).

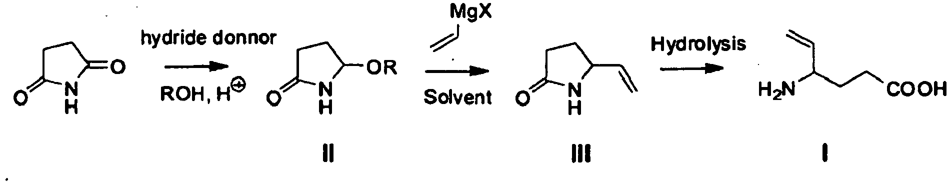

Succinimide, or pyrrolidine-2,5-dione according to the IUPAC nomenclature, is a cyclic imide widely used in commercial drugs. The hydride donor is usually an aluminium hydride complex or a boron hydride complex such as lithium aluminium hydride (LiAlH₄) or sodium borohydride (NaBH₄). The alcoholic solvent of step a) is a primary, secondary or tertiary alcohol. In a preferred embodiment, the alcoholic solvent is selected from methanol or ethanol. The protic acid is a liquid, a solid eventually in solution or a gas. In a particular embodiment, the protic acid is selected from acetic acid, sulfuric acid, tartaric acid, hydrogen chloride, nitric acid, boric acid, citric acid, or lactic acid. In a preferred embodiment, the protic acid is hydrogen chloride.

The vinyl magnesium halide reagent of step b) is selected from vinyl magnesium chloride, vinyl magnesium bromide and vinyl magnesium iodide. In a preferred embodiment the vinyl magnesium halide reagent is vinyl magnesium chloride. The solvent of step b) is any solvent known by one skilled in the art that is suitable for a Grignard reaction such as diethyl ether, toluene, tetrahydrofuran (THF) or methyl-2-tetrahydrofuran (MeTHF) or a mixture thereof. The present invention further describes a process as detailed above, where the vinyl magnesium halide reagent of step b) is present in an amount comprised between 2 and 3 molar equivalents. In a preferred embodiment, the vinyl magnesium halide reagent of step b) is present in the amount of 2 to 2,5 equivalents.

The hydrolysis of step c) can be carried out using techniques known by one skilled in the art such as potassium hydroxide (see EP 0427197, US 4,621,145 or US 6,090,979) or sodium hydroxide or aqueous acidic hydrolysis.

In a particular embodiment, the invention provides a process for preparing 4-amino-5-hexenoic acid (I) according to the above-mentioned process, that comprises preparing 5-vinyl-2-pyrrolidone (III) according to steps a) to b) above, the steps being undertaken without isolation of the 5-alkoxy-2-pyrrolidone intermediate (II), and then hydrolyzing 5-vinyl-2-pyrrolidone (III) to 4-amino-5-hexenoic acid (I) in a subsequent step. This embodiment has the advantage of decreasing the global duration of the preparation process by deleting the isolation of the intermediate (II). Consequently, the invention further provides a process as defined above wherein steps a) and b) are being undertaken without isolation of the 5-alkoxy-2-pyrrolidone intermediate.

In yet another particular embodiment, the invention provides a process for preparing 4-amino-5-hexenoic acid (I) according to steps a) to c) above, the steps being undertaken without isolation of the 5-alkoxy-2-pyrrolidone (II) or 5-vinyl-2-pyrrolidone (III) intermediates. This embodiment has the advantage of decreasing the global duration of the preparation process by deleting the isolation of the intermediates (II) and (III). Consequently the present invention further provides a process as defined above wherein steps a), b) and c) are being undertaken without isolation of the 5-alkoxy-2-pyrrolidone or 5-vinyl-2-pyrrolidone intermediates.

In yet another embodiment, the invention provides a process for preparing 4-amino-5-hexenoic acid (I), which comprises:
a) reducing succinimide with a hydride donor in an alcoholic solvent in presence of a protic acid to produce a 5-alkoxy-2-pyrrolidone intermediate (II);
b) submitting said 5-alkoxy-2-pyrrolidone intermediate (II) to i) one equivalent of a basic reagent, so as to deprotonate the lactame labile hydrogen, and ii) at least one equivalent of a vinyl magnesium halide reagent (vinyl Grignard reagent), to form 5-vinyl-2-pyrrolidone (III), the two steps being undertaken in the same reaction vessel; and
c) hydrolyzing said 5-vinyl-2-pyrrolidone (III) to 4-amino-5-hexenoic (I).

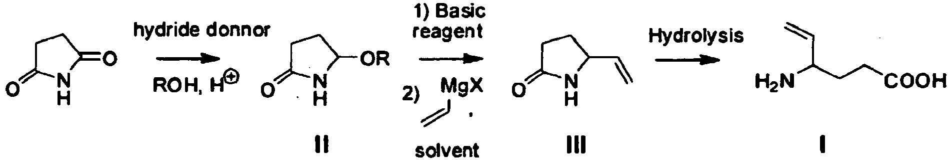

The succinimide, hydride donor, alcoholic solvent and protic acid are as defined above in the first embodiment.

The vinyl magnesium halide reagent is as defined above in the first embodiment. The basic reagent is any reagent that is able to accept hydrogen ions or more generally to donate electron pairs as it is known by one skilled in the art. Suitable basic reagents are ethylmagnesium bromide (EtMgBr), isopropanolmagnesium bromide (ⁱPrMgBr), butyllithium (BuLi) and methylmagnesium chloride (MeMgCl) as examples. In a particular embodiment the basic reagent of step b) is present in an amount comprised between 0.5 to 1.5 molar equivalents (eq.). In yet another particular embodiment the vinyl magnesium halide reagent of step b) is present in an amount comprised between 1 to 2 molar equivalents (eq.). In a preferred embodiment, the basic reagent of step b) is present in the amount of 1.0 molar equivalent (eq.) and the vinyl magnesium halide reagent of step b) is present in the amount of 1.5 equivalents.

The step c) is as defined above in the first embodiment.

This preparation process has the advantage of reducing the global synthesis cost, as vinyl magnesium halides are moderately expensive reagents, and that the base used for the deprotonation step are cheaper than vinyl magnesium halides.

In a further embodiment, the invention provides a process for preparing 4-amino-5-hexenoic acid (I) as detailed above, wherein the steps a) and b) are being undertaken without isolation of the 5-alkoxy-2-pyrrolidone intermediate (II), and then hydrolyzing 5-vinyl-2-pyrrolidone (III) to 4-amino-5-hexenoic acid (I) in a subsequent step. This embodiment has the advantage of decreasing the global duration of the preparation process by deleting the isolation of the intermediate (II). Consequently, the invention further provides a process as defined above wherein steps a) and b) are being undertaken without isolation of the 5-alkoxy-2-pyrrolidone intermediate (II).

In yet another embodiment, the invention provides a process for preparing 4-amino-5-hexenoic acid (I) according to steps a) to c) above, the steps being undertaken without isolation of the 5-alkoxy-2-pyrrolidone (II) or 5-vinyl-2-pyrrolidone (III) intermediates. This embodiment has the advantage of decreasing the global duration of the preparation process by deleting the isolation of the intermediates (II) and (III). Consequently the present invention further provides a process as defined above wherein steps a), b) and c) are being undertaken without isolation of the 5-alkoxy-2-pyrrolidone or 5-vinyl-2-pyrrolidone intermediates.

As described above, 4-amino-5-hexenoic acid is known to be a gamma-aminobutyric transaminase (GABA-T) inhibitor. GABA-T is the enzyme responsible for the catabolism of gamma-aminobutyric acid (GABA) and its over functioning in the brain increases the GABA level and this is involved in pathologies and syndromes among which epilepsy and related syndromes.

Examples of relevant pathologies and symptoms are epilepsy and especially complex partial seizures, secondary generalized seizures and infantile spasms in West syndrome.

The following examples illustrate the new preparation process according to the invention.

### Examples

The following steps refer to the preparation process of the invention as described above.

### Step a) Reduction of succinimide:

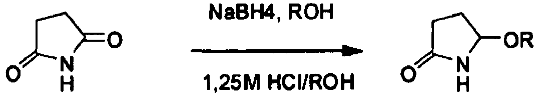

To a solution of 10 g of succinimide (100 mmol, 1 eq.) in ethanol (300 ml) at -10°C and under argon atmosphere, sodium borohydride (5.75 g, 150 mmol, 1.5 eq.) is added by portion. To the reaction mixture is added every 15 minutes, 2 ml of a 1.25 M solution of hydrogen chloride in ethanol (Vₜₒₜ = 33 ml). After 4 h of agitation, 110 ml of a 1.25 M solution of hydrogen chloride in ethanol is added and the reaction mixture is stirred for 2 h at -5°C. Then, 4 ml of a saturated potassium hydroxide solution in ethanol was added and the solvent is partially removed. The crude reaction mixture is filtered over celite and washed with CHCl₃ (4x50 ml). The organic phase is washed with water (1x50 ml) and dried over Na₂SO₄. Filtration and vacuum distillation afford 5-ethoxy-2-pyrrolidone as a white powder (10.4 g, 80%). 5-ethoxy-2-pyrrolidone (1 g.) can be recrystallized from hexanes to produce white needles (0.7 g., 70% recrystallization yield).

Other step a) examples with various experimental conditions are summarized in the following table 1. The group R in the above formula is different according to the reaction conditions. In the examples below R is methyl or ethyl.

**Table 1: intermediate products obtained after step a) of the new preparation process**

| Solvent | R | NaBH₄ | Acid | Reaction conditions | Yield |
|---|---|---|---|---|---|
| Methanol | Methyl | 2.5 eq. | 0.2 ml 1.25 M HCl in MeOH | 4h at 0°C then RT / overnight | 30% |
| Ethanol | Ethyl | 2.5 eq. | 0.3 ml 1.25 M HCl in EtOH | 4h at 0°C then RT / overnight | 45% |
| Ethanol | Ethyl | 1.5 eq. | 0.6 ml 1.25 M HCl in EtOH | 4h at -10°C | 84% |

The intermediate product is ready for step b).

A global yield of 30 % is obtained with R being methyl, the alcoholic solvent being methanol, the hybrid donor being NaBH₄ in an amount of 2.5 eq., the protic acid being HCl (0.2 ml at 1.25 M) in MeOH and the reaction conditions being 4 hours at 0°C and then at room temperature overnight.

A global yield of 45 % is obtained with R being ethyl, the alcoholic solvent being ethanol, the hybrid donor being NaBH₄ in an amount of 2.5 eq., the protic acid being HCl (0.3 ml at 1.25 M) in EtOH and the reaction conditions being 4 hours at 0°C and then at room temperature overnight.

A better global yield of 84 % is obtained with R being ethyl, the alcoholic solvent being ethanol, the hybrid donor being NaBH₄ in an amount of 1.5 eq., the protic acid being HCl (0.6 ml at 1.25 M) in EtOH and the reaction conditions being 4 hours at -10°C.

### Step b) reaction of 5-alkoxy-2-pyrrolidones with vinyl magnesium halides:

5-vinyl-2-pyrrolidone (III) can be obtained in high yields by reaction of 5-alkoxy-2-pyrrolidones with vinyl magnesium halides, as described below.

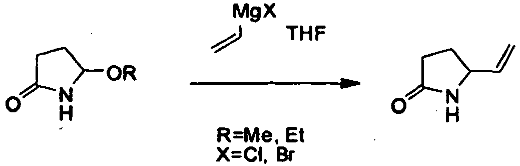

To a solution of 1 g of 5-ethoxy-2-pyrrolidone (7.75 mmol, 1 eq.) in anhydrous THF (3 ml) at 0°C and under argon atmosphere, 12.2 ml (15.6 mmol, 2 eq.) of vinyl magnesium chloride (1.6 M in THF) is added dropwise. The resulting mixture is refluxed for 2.5 h. The solution is cooled at room temperature and 10 ml of NH₄Cl is added. The aqueous phase is extracted with THF (1x20 ml) and ethyl acetate (2x20 ml). The organic layers are washed with brine (1x20 ml) and dried over Na₂SO₄. Filtration and vacuum distillation afford compound 5-vinyl-2-pyrrolidone (III) as a colorless oil (560 mg, 65%).

Examples of reactions according to step b) with various reaction parameters are summarized in the following table 2.

**Table 2:**

| R | Grignard reagent | Solvent (concentration) | Conditions | Yield |
|---|---|---|---|---|
| Methyl | Vinyl magnesium chloride (2 eq.) | THF [0.3 M] | 0°C (addition), then reflux 3 h | 60% |
| Ethyl | Vinyl magnesium chloride (2 eq.) | THF [0.3 M] | 0°C (addition), then reflux 2.5 h | 65% |
| Ethyl | Vinyl magnesium bromide (2 eq.) | THF [0.4 M] | 0°C (addition), then reflux 3 h | 81% |
| Ethyl | Vinyl magnesium bromide (2 eq.) | MeTHF [0.4 M] | 0°C (addition), then reflux 3 h | 23% |

A satisfying global yield of 60% is obtained with R being methyl, the Grignard reagent being vinyl magnesium chloride in an amount of 2 equivalents, the solvent being THF in an amount of 0.3 M at 0°C and then reflux for 3h.

A satisfying global yield of 65% is obtained with R being ethyl, the Grignard reagent being vinyl magnesium chloride in an amount of 2 equivalents, the solvent being THF in an amount of 0.3 M at 0°C and then reflux for 3h.

A better global yield of 81% is obtained with R being ethyl, the Grignard reagent being vinyl magnesium bromide in an amount of 2 equivalents, the solvent being MeTHF in an amount of 0.4 M.

Alternatively, 5-vinyl-2-pyrrolidone (III) could be obtained in high yields by first reacting 5-alkoxy-2-pyrrolidones (II) with one equivalent of a base, then with at least one equivalent of a vinyl magnesium halide, as described below. This procedure has the advantage of reducing the synthesis cost, as vinyl magnesium halides are moderately expensive reagents, and that the base used for the deprotonation step are cheaper than vinyl magnesium halides.

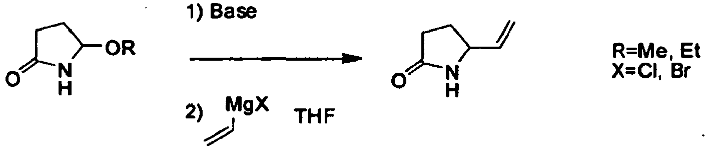

To a solution of 1 g of 5-ethoxy-2-pyrrolidone (7.75 mmol, 1 eq.) in anhydrous THF (3 ml) at 0°C and under argon atmosphere, 2.60 ml of methylmagnesium chloride (7.75 mmol, 1 eq.) in THF is added dropwise. The solution is stirred for 30 minutes at - 15°C. Then, 7.3 ml (11.65 mmol, 1.5 eq.) of vinyl magnesium chloride (1.6 M in THF) is added, the resulting mixture is refluxed for 2 h. The solution is cooled at room temperature and 10 ml of NH₄Cl is added. The aqueous phase is extracted with THF (1x20 ml) and ethyl acetate (2x20 ml). The organic layers is washed with brine (1x20 ml) and dried over Na₂SO₄. Filtration and vacuum distillation afford compound 5-vinyl-2-pyrrolidone (III) as a colorless oil (757 mg, 88%).

Examples of various reaction parameters are summarized in the following table 3.

**Table 3:**

| R | Solvent | Base | Grignard reagent | Yield |
|---|---|---|---|---|
| Ethyl | MeTHF | EtMgBr (1 M/THF, 1 eq.) 30 min, 0°C | Vinyl magnesium chloride (1.6 M/THF, 1.5 eq.) 2h, reflux | 80% |
| Ethyl | THF | EtMgBr (1 M/THF, 1 eq.) 30 min, -15°C | Vinyl magnesium chloride (1.6 M/THF, 1.5 eq.) 2h, reflux | 93% |
| Ethyl | Toluene | EtMgBr (1 M/THF, 1 eq.) 30 min, 0°C | Vinyl magnesium chloride (1.6 M/THF, 1.5 eq.) 2h, reflux | 59% |
| Ethyl | THF | NaH (1 eq.) 30 min, 0°C | Vinyl magnesium chloride (1.6 M/THF, 1 eq.) 2h, reflux | 30% |
| Ethyl | THF | ⁱPrMgBr (M/THF, 1 eq.) 30 min, 0°C | Vinyl magnesium chloride (1.6 M/THF, 1.5 eq.) 2h, reflux | 77% |
| Ethyl | THF | BuLi (1.8 M/pentane, 1 eq.) 30 min, -40°C | Vinyl magnesium chloride (1.6 M/THF, 1.5 eq.) 2h, reflux | 70% |
| Ethyl | MeTHF | MeMgCl (3M/THF, 1eq.) 30min, 0°C | Vinyl magnesium chloride (1F, 1.5 eq.) 2h, reflux | 90% |

The better global yields are comprised between 80 % and 90 %.

A yield of 80 % is obtained with R being ethyl, the solvent being MeTHF, the Grignard reagent being vinyl magnesium chloride (1.6 M/THF, 1.5 eq., 2 hours, reflux), and the basis reagent being EtMgBr (1 M/THF, 1 eq., 30 min, 0°C).

A yield of 90 % is obtained with R being ethyl, the solvent being MeTHF, the Grignard reagent being vinyl magnesium chloride (1.6 M/THF, 1.5 eq., 2 hours, reflux), and the basic reagent being MeMgCl (3 M/THF, 1 eq., 30min, 0°C).

A yield of 93 % is obtained with R being ethyl, the solvent being THF, the Grignard reagent being vinyl magnesium chloride (1.6 M/THF, 1.5 eq., 2 hours, reflux), and the basic reagent being EtMgBr (1 M/THF, 1 eq., 30 min, -15°C).

### Step c) 5-vinyl-2-pyrrolidone (III) hydrolysis to 4-amino-5-hexenoic acid (I):

5-vinyl-2-pyrrolidone (III) can be hydrolyzed to 4-amino-5-hexenoic acid of formula (I), according to one of the two procedures described in US Pat. 4,621,145 or US Pat. 6,090,979.

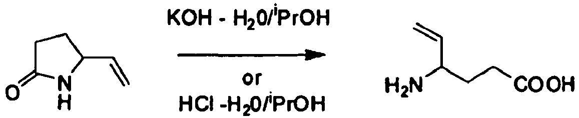

Under argon, 1 g of 5-vinyl-2-pyrrolidone (6 mmol) is dissolved in a mixture of isopropanol (10 ml) and deionized water (1 ml). To this solution, 0.9 g of potassium hydroxide is added at room temperature and the mixture is then heated at 60°C for 16 h. After cooling, the reaction is quenched with 1 ml of glacial acetic acid and crude 4-amino-5-hexenoic acid precipitates in the reaction mixture. 4-amino-5-hexenoic acid is recovered by filtration and recrystallized in a mixture of water/isopropanol to give 700 mg of white 4-amino-5-hexenoic acid crystals (60 % yield).

### Other embodiment: combination of steps a) and b)

Steps a) and b) as detailed above can be combined without isolating the intermediate 5-alkoxy-2-pyrrolidone (II).

In a reaction vessel under argon, succinimide (2 g, 20 mmol) is dissolved in anhydrous ethanol (60 ml), then cooled to 5°C. NaBH₄ (1.15 g, 30 mmol) is added portion wise over 2 minutes. Hydrochloric acid (1.25 M) in anhydrous ethanol (30 ml) is then added over a period of 2.5 h and the mixture is agitated 3 h at -5°C. The reaction mixture is quenched with a saturated solution of potassium hydroxide in ethanol, until pH = 7 to 8 is reached. Ethanol is partially evaporated, and the reaction mixture filtered over a celite bed. Celite is washed with 2-MeTHF (2x50 ml), then the organic phases are gathered, washed with 30 ml of saturated brine and dried over Na₂SO₄. The organic phase is evaporated twice under reduced pressure to remove 2-MeTHF and residual ethanol. Dry 2-MeTHF is added under argon (50 ml), cooled at 0°C, then ethyl magnesium bromide (1 M in THF, 20 ml, 1 eq.) and the reaction mixture are agitated for 30 minutes at 0°C. Vinyl magnesium chloride (1.6 M in THF, 20 ml, 1.6 eq.) is added at 0°C and the reaction mixture is refluxed for 2 hours.

After 2 hours, the reaction mixture is cooled at room temperature, quenched with a saturated NH₄Cl solution, and extracted twice with methylene chloride (2x30 ml). Organic phases are gathered, rinsed with saturated brine (20 ml), dried over Na₂SO₄, filtered and concentrated under reduced pressure to give 5-vinyl-2-pyrrolidone as slightly yellow oil (555 mg, 25% yield).

## Claims

1. A process for producing 4-amino-5-hexenoic acid using succinimide as raw material, **characterized in that** it comprises at least a first step a) consisting of reducing said succinimide with a hydride donor in an alcoholic solvent in presence of a protic acid to produce a 5-alkoxy-2-pyrrolidone intermediate.

2. A process according to claim 1 which further comprises the following steps:
b) reacting said 5-alkoxy-2-pyrrolidone intermediate with at least 2 equivalents of a vinyl magnesium halide reagent (vinyl Grignard reagent) in a suitable solvent, to form 5-vinyl-2-pyrrolidone; and
c) hydrolyzing said 5-vinyl-2-pyrrolidone to form 4-amino-5-hexenoic acid.

3. A process according to claim 2, where the vinyl magnesium halide reagent of step b) is present in the amount of 2 to 3 molar equivalents.

4. A process according to claim 2, where the vinyl magnesium halide reagent of step b) is present in the amount of 2 to 2,5 molar equivalents.

5. A process according to claim 2 wherein steps a) and b) are being undertaken without isolation of the 5-alkoxy-2-pyrrolidone intermediate.

6. A process according to claim 2 wherein steps a), b) and c) are being undertaken without isolation of the 5-alkoxy-2-pyrrolidone or 5-vinyl-2-pyrrolidone intermediates.

7. A process according to claim 1 which further comprises the following steps:
b) submitting said 5-alkoxy-2-pyrrolidone intermediate to i) one equivalent of a basic reagent in a suitable solvent, and ii) at least one equivalent of a vinyl magnesium halide reagent (vinyl Grignard reagent), to form 5-vinyl-2-pyrrolidone, the two steps being undertaken in the same reaction vessel, and
c) hydrolyzing said 5-vinyl-2-pyrrolidone to 4-amino-5-hexenoic acid.

8. A process according to claim 7, where the basic reagent of step b) is present in an amount comprised between 0.5 to 1.5 molar equivalents.

9. A process according to claim 7, where the vinyl magnesium halide reagent of step b) is present in an amount comprised between 1.0 to 2.0 molar equivalents.

10. A process according to claim 7, wherein steps a) and b) are being undertaken without isolation of the 5-alkoxy-2-pyrrolidone intermediate.

11. A process according to claim 7, wherein steps a), b) and c) are being undertaken without isolation of the 5-alkoxy-2-pyrrolidone or 5-vinyl-2-pyrrolidone intermediates.

## Patentansprüche

1. Verfahren zum Herstellen von 4-Amino-5-hexensäure unter Verwendung von Succinimid als Ausgangsmaterial, **dadurch gekennzeichnet, dass** es wenigstens einen ersten Schritt a) umfasst, der aus der Reduktion des genannten Succinimids mit einem Hydriddonor in einem alkoholischen Lösemittel in Anwesenheit einer protischen Säure besteht, um ein 5-Alkoxy-2-pyrrolidonintermed zu bilden.

2. Verfahren nach Anspruch 1, das ferner die folgenden Schritte umfasst:
b) Reaktion des genannten 5-Alkoxy-2-pyrrolidonintermediats mit wenigstens zwei Äquivalenten Vinylmagnesiumhalogenidreagenz (Vinyl-Grignard-Reagenz) in einem geeigneten Lösemittel, um 5-Vinyl-2-pyrrolidon zu bilden; und
c) Hydrolysieren des genannten 5-Vinyl-2-pyrrolidons, um 4-Amino-5-hexensäure zu bilden.

3. Verfahren nach Anspruch 2, wobei das Vinylmagnesiumhalogenidreagenz von Schritt b) in der Menge von 2 bis 3 Molaräquivalenten vorhanden ist.

4. Verfahren nach Anspruch 2, wobei das Vinylmagnesiumhalogenidreagenz von Schritt b) in der Menge von 2 bis 2,5 Molaräquivalenten vorhanden ist.

5. Verfahren nach Anspruch 2, wobei Schritte a) und b) ohne Isolierung des 5-Alkoxy-2-pyrrolidonintermediats vorgenommen werden.

6. Verfahren nach Anspruch 2, wobei Schritte a), b) und c) ohne Isolierung des 5-Alkoxy-2-pyrrolidon- oder 5-vinyl-2-pyrrolidonintermediats vorgenommen werden.

7. Verfahren nach Anspruch 1, das ferner die folgenden Schritte umfasst:
b) Zugaben des genannten 5-Alkoxy-2-pyrrolidonintermediats zu i) einem Äquivalent eines basischen Reagenz in einem geeigneten Lösemittel und ii) wenigstens einem Äquivalent eines Vinylmagnesiumhalogenidreagenz (Vinyl-Grignard-Reagenz), um 5-Vinyl-2-pyrrolidon zu bilden, wobei die beiden Schritte in dem gleichen Reaktionsgefäß vollzogen werden, und
c) Hydrolysieren des genannten 5-vinyl-2-pyrrolidons zu 4-Amino-5-hexensäure.

8. Verfahren nach Anspruch 7, wobei das basische Reagenz von Schritt b) in einer Menge vorhanden ist, die zwischen 0, 5 bis 1,5 Molaräquivalente umfasst.

9. Verfahren nach Anspruch 7, wobei das Vinylmagnesiumhalogenid-reagenz von Schritt b) in einer Menge vorhanden ist, die zwischen 1,0 bis 2,0 Molaräquivalente umfasst.

10. Verfahren nach Anspruch 7, wobei Schritte a) und b) ohne Isolierung des 5-Alkoxy-2-pyrrolidonintermediats vorgenommen werden.

11. Verfahren nach Anspruch 7, wobei Schritte a), b) und c) ohne Isolierung des 5-Alkoxy-2-pyrrolidon- oder 5-Vinyl-2-pyrrolidonintermediats vorgenommen werden.

## Revendications

1. Procédé de production d'acide 4-amino-5-hexenoic utilisant un succinimide en tant que matière première, **caractérisé en ce qu'**il comprend au moins une première étape a) qui consiste à réduire ledit succinimide avec un donneur d'hydrures dans un solvant alcoolique en présence d'un acide protique pour produire un intermédiaire 5-alkoxy-2-pyrrolidone.

2. Procédé selon la revendication 1 qui comprend en outre les étapes suivantes:
b) faire réagir ledit intermédiaire 5-alkoxy-2-pyrrolidone avec au moins 2 équivalents d'un réactif halogénure vinylmagnésium (réactif vinyle de Grignard) dans un solvant adéquat, pour former un 5-vinyl-2-pyrrolidone; et
c) hydrolyser ledit 5-vinyl-2-pyrrolidone en acide 4-amino-5-hexenoic.

3. Procédé selon la revendication 2, dans lequel le réactif halogénure vinylmagnésium de l'étape b) est présent en une quantité comprise entre 2 et 3 équivalents molaires.

4. Procédé selon la revendication 2, dans lequel le réactif halogénure vinylmagnésium de l'étape b) est présent en une quantité comprise entre 2 et 2,5 équivalents molaires.

5. Procédé selon la revendication 2, dans lequel les étapes a) et b) sont entreprises sans isolement de l'intermédiaire 5-alkoxy-2-pyrrolidone.

6. Procédé selon la revendication 2 dans lequel les étapes a), b) et c) sont entreprises sans isolement des intermédiaires 5-alkoxy-2-pyrrolidone ou 5-vinyl-2-pyrrolidone.

7. Procédé selon la revendication 1 qui comprend en outre les étapes suivantes :
b) soumettre l'intermédiaire 5-alkoxy-2-pyrrolidone à i) un équivalent d'un réactif basique dans un solvant adéquat, et ii) au moins un équivalent d'un réactif halogénure de vinylmagnésium (réactif vinyle de Grignard), pour former un 5-vinyl-2-pyrrolidone, les deux étapes étant entreprises dans le même récipient, et
c) hydrolyser ledit 5-vinyl-2-pyrrolidone en acide 4-amino-5-hexenoic.

8. Procédé selon la revendication 7, dans lequel le réactif basique de l'étape b) est présent en une quantité comprise entre 0,5 et 1,5 équivalents molaires.

9. Procédé selon la revendication 7, dans lequel le réactif halogénure de vinylmagnésium de l'étape b) est présent en une quantité comprise entre 1,0 et 2,0 équivalents molaires.

10. Procédé selon la revendication 7, dans lequel les étapes a) et b) sont entreprises sans isolement de l'intermédiaire 5-alkoxy-2-pyrrolidone.

11. Procédé selon la revendication 7, dans lequel les étapes a), b) et c) sont entreprises sans isolement des intermédiaires 5-alkoxy-2-pyrrolidone ou 5-vinyl-2-pyrrolidone.
